# EUROPÄISCHE PATENTSCHRIFT

(11) **EP 2 653 119 B1**
(45) Veröffentlichungstag und Bekanntmachung des Hinweises auf die Patenterteilung: **11.10.2017**
(21) Anmeldenummer: 13001769.2
(22) Anmeldetag: 06.04.2013
(51) Int. Cl.: A61B 17/29, A61B 17/295, A61B 17/32, A61B 17/00, A61B 18/14

(54) **Medizinisches Instrument und Verfahren zum Zusammensetzen eines medizinischen Instruments**
Medical instrument and method for assembling a medical instrument
Instrument médical et procédé d'assemblage d'un instrument médical

(30) Priorität: 18.04.2012 DE 102012007653
(43) Veröffentlichungstag der Anmeldung: 23.10.2013
(73) Patentinhaber: Karl Storz GmbH & Co. KG, 78532 Tuttlingen (DE)
(72) Erfinder: Bacher, Uwe, 78532 Tuttlingen (DE); Merz, Robin, 78532 Tuttlingen (DE); Schneider, Sven, 78532 Tuttlingen (DE); Stefan, Jochen, 78532 Tuttlingen (DE)

(56) Entgegenhaltungen:
- WO-A1-00/54659
- WO-A1-98/01080
- WO-A1-99/58066
- WO-A2-2009/132359
- DE-A1- 4 445 105
- DE-U1- 9 320 450
- US-A- 6 059 719
- US-A1- 2003 114 839
- US-A1- 2008 021 278
- US-A1- 2009 234 378
- US-A1- 2010 121 141
- US-A1- 2011 276 048
- US-B1- 6 358 267

## Beschreibung

Die vorliegende Erfindung ist auf ein medizinisches Instrument mit einem Außenschaft, einer Übertragungseinrichtung und einem Werkzeug und auf ein Verfahren zum Zusammensetzen eines Werkzeugs, einer Übertragungseinrichtung und eines Außenschafts zu einem medizinischen Instrument gerichtet.

Die Erwartungen an medizinische Instrumente, insbesondere an medizinische Instrumente für mikroinvasive Eingriffe steigen beständig. Bereits in Vielfalt angeboten und weit verbreitet sind medizinische Instrumente mit einem Werkzeug mit Greif- oder Schneidefunktion am distalen Ende. Zunehmend kommen weitere Funktionen und Freiheitgrade hinzu, beispielsweise eine Rotation des Werkzeugs um die Längsachse des Schafts, eine Abwinkelbarkeit des Schafts proximal des Werkzeugs oder eine zweite, unabhängig steuerbare Wirkeinrichtung am Werkzeug. Zur Steuerung dieser weiteren Funktionen oder Freiheitsgrade kann ein zweites Übertragungselement im Schaft des medizinischen Instruments vorgesehen sein, beispielsweise eine zweite Übertragungsstange.

Bei mehrfach verwendbaren medizinischen Instrumenten ist eine möglichst weitgehende Zerlegbarkeit als Voraussetzung für eine vollständige Reinigung von besonderer Bedeutung. Dabei ist zu beobachten, dass eine wachsende Anzahl von Funktionen und Freiheitsgraden eine immer weitergehende Zerlegbarkeit bzw. eine Zerlegbarkeit in immer mehr Einzelteile bedingt. Gleichzeitig sollen die Handhabung aller Einzelteile, das Zerlegen und Zusammensetzen des medizinischen Instruments möglichst sicher, einfach und intuitiv bzw. mit geringem Schulungsaufwand möglich sein. Insbesondere soll ein Zerlegen mit möglichst wenigen Handgriffen möglich sein. Dabei ist es hilfreich, wenn das medizinische Instrument möglichst wenige Betätigungseinrichtungen zur Entriegelung von mechanischen Verbindungen von Einzelteilen aufweist.

Die Schrift DE4445105A1 offenbart ein Instrument und ein Verfahren entsprechend dem Oberbegriff der Ansprüche 1 und 13, es beschreibt ein Operationselement mit einem Außenschaft, darin einer Übertragungseinrichtung zum Übertragen einer Kraft auf ein Werkzeug. Der Außenschaft ist über eine Kupplungseinrichtung lösbar mit dem Werkzeug verbunden. Die Übertragungseinrichtung ist ebenso über eine Kupplungseinrichtung mittels Verschraubung lösbar mit dem Werkzeug verbunden.

Das Patent US 6358267 B1 zeigt ein medizinisches Instrument, mit einem Außenschaft; einer Übertragungseinrichtung zum Übertragen zumindest einer Kraft; einem Werkzeug mit einer Kupplung zum lösbaren mechanischen Verbinden des Werkzeugs mit dem Außenschafts und mit einer weiteren Kupplung, um das Werkzeug lösbar mechanisch mit der Übertragungseinrichtung zu verbinden. Dabei sind die Verbindung Werkzeug-Außenschaft durch eine Bajonett-Verbindung und die Verbindung Werkzeug- Übertragungseinrichtung durch eine Schraubverbindung realisiert. Die Druckschrift US 2008/021278 A1 offenbart eine chirurgische Vorrichtung mit einem Werkzeug und einem System zum Verbinden des Werkzeugs mit Außenschaft wie auch Übertragungselement. Als Verbindungstechniken werden dabei eine Bajonett-Verbindung und eine Schraubverbindung vorgestellt.

Die Patentanmeldung WO 2009/132359 A2 behandelt eine chirurgische Vorrichtung mit Verbindungssystem zum Verbinden einer Griff-/Welle-/Betätigungsstangeneinheit mit dem distalen Werkzeug. Das Verbindungssystem umfasst zur Verbindung mit dem Werkzeug eine Kugel an der Griff-/Welle-/Betätigungsstangeneinheit und zur Verbindung mit dem Außenschaft eine Schraubverbindung.

WO 98/01080 A1 stellt ein endoskopisches chirurgisches Instrument vor, das eine Griffanordnung, eine Schaft aus zwei Teilen, nämlich einem längeren aus einer Spirale geformten Teil und einem kürzeren aus einer Spirale geformten Teil, sowie ein Übertragungselement, ebenso aus einem längeren und einem kürzeren Teil, umfasst. Als Kupplungen von Schaft und Übertragungselement zum Werkzeug sind beispielsweise eine Kugel, passend zu einer Kugelaufnahme, und auch ein Bajonettverschluss vorgestellt.

Die Schrift US 2003/114839 A1 behandelt eine chirurgische Vorrichtung, wiederum mit einem Handgriff, einem Werkzeug, einer Übertragungsstange und einem Außenschaft. Als Verbindungstechniken zwischen Werkzeug und Außenschaft oder auch Übertragungsstange sind beispielsweise Gewinde, Drehschlösser vorgestellt.

Das Gebrauchsmuster DE9320450U1_beschreibt eine medizinische, insbesondere endoskopische, Zange mit einem Handstück, einem Außenrohr, einer in dem Außenrohr verschiebbaren Zugstange und einem Werkzeug. Werkzeug und Zugstange sind dabei mittels eines Bajonettverschluss' verbunden. Dieser Bajonettverschluss ist bezüglich dem Außenrohr axial verschieblich und bewirkt bei Manipulation des Handstücks, dass das Werkzeug betätigt wird.

Die Patentanmeldung US 2011/0276048 A1 zeigt eine Zange mit einem Gehäuse, einem Schaft und einem Werkzeugaufbau mit zwei Maulteilen sowie einem axial bewegbaren Messer. Die verschieden Bauteile sind alle miteinander verbunden, die Verbindungarten jedoch nicht eingehend beschrieben.

Eine Aufgabe der vorliegenden Erfindung besteht darin, ein verbessertes medizinisches Instrument mit einem Außenschaft, einer Übertragungseinrichtung und einem Werkzeug und ein verbessertes Verfahren zum Zusammensetzen eines Werkzeugs, einer Übertragungseinrichtung und eines Außenschafts zu einem medizinischen Instrument zu schaffen. Die zu lösende Aufgabe wird durch ein erfindungsgemäßes Instrument und Verfahren entsprechend den Ansprüchen 1 und 13 gelöst, besondere Ausführungsformen sind in den Unteransprüchen angegeben.

Ein medizinisches Instrument umfasst einen Außenschaft, eine im Außenschaft anzuordnende Übertragungseinrichtung zum Übertragen zumindest entweder einer Kraft oder eines Drehmoments und ein Werkzeug mit einer Außenschaft-Kupplungseinrichtung zum lösbaren mechanischen Verbinden des Werkzeugs mit einer Werkzeug-Kupplungseinrichtung am distalen Ende des Außenschafts und mit einer Übertragungs-Kupplungseinrichtung zum lösbaren mechanischen Verbinden des Werkzeugs mit einer Werkzeug-Kupplungseinrichtung am distalen Ende der Übertragungseinrichtung, wobei die mechanische Verbindung der Werkzeug-Kupplungseinrichtung am Außenschaft mit der Außenschaft-Kupplungseinrichtung am Werkzeug durch eine Bewegung des Außenschafts in einer ersten Richtung relativ zum Werkzeug herstellbar ist, wobei die mechanische Verbindung der Werkzeug-Kupplungseinrichtung an der Übertragungseinrichtung mit der Übertragungs-Kupplungseinrichtung am Werkzeug durch eine Bewegung der Übertragungseinrichtung in einer zweiten Richtung relativ zum Werkzeug herstellbar ist, und wobei die erste Richtung entgegengesetzt zur zweiten Richtung ist. Das medizinische Instrument ist insbesondere ein mikroinvasiv-chirurgisches Instrument. Das medizinische Instrument kann zusätzlich zu dem Außenschaft, der Übertragungseinrichtung und dem Werkzeug weitere Bestandteile aufweisen oder zur Kombination bzw. zum Zusammensetzen mit weiteren Bestandteilen vorgesehen sein. Insbesondere kann das medizinische Instrument eine Handhabungseinrichtung umfassen oder zum Zusammensetzen mit einer Handhabungseinrichtung vorgesehen und ausgebildet sein.

Der Außenschaft kann gerade oder gekrümmt, starr oder flexibel sein. Die Übertragungseinrichtung ist beispielsweise eine Übertragungsstange oder ein Innenschaft, wobei in einem im Wesentlichen rohrförmigen Innenschaft eine weitere Übertragungseinrichtung in Form einer Übertragungsstange angeordnet sein kann. Die Übertragungseinrichtung kann ihrerseits gerade oder gekrümmt, starr oder flexibel sein. Wenn der Außenschaft gekrümmt oder flexibel ist, ist die Übertragungseinrichtung insbesondere zumindest teilweise flexibel, um in dem gekrümmten Außenschaft bewegt, insbesondere verschoben und/oder rotiert werden zu können.

Das Werkzeug weist insbesondere mehrere Funktionen auf, die mittels mehrerer Übertragungseinrichtungen unabhängig voneinander gesteuert werden können. Beispielsweise werden eine erste Funktion des Werkzeugs mittels einer Übertragungsstange und eine zweite Funktion des Werkzeugs mittels eines Innenschafts gesteuert.

Die erste Richtung und die zweite Richtung sind insbesondere entgegengesetzte translatorische oder entgegengesetzte rotatorische Richtungen. Durch die lösbare mechanische Kopplung des Werkzeugs einerseits mit dem Außenschaft und andererseits mit der Übertragungseinrichtung mit Bewegungen in entgegengesetzten Richtungen können beide mechanische Verbindungen verriegelt werden, indem Außenschaft und Übertragungseinrichtung hinsichtlich einer Relativbewegung parallel zur ersten und zweiten Richtung miteinander verriegelt werden. In einem alternativen Beispiel, welches nicht Teil der vorliegenden Erfindung ist, ist die erste Richtung nicht-parallel zur zweiten Richtung, insbesondere senkrecht zur zweiten Richtung. Eine Rotationsbewegung ist insofern senkrecht zu einer Translationsbewegung entlang der Rotationsachse der Rotationsbewegung, als bei einer Rotationsbewegung - von den Punkten auf der Rotationsachse abgesehen - alle Punkte jederzeit in Richtungen senkrecht zur Rotationsachse bewegt werden. Beispielsweise sind die mechanischen Verbindung der Werkzeug-Kupplungseinrichtung am Außenschaft mit der Außenschaft-Kupplungseinrichtung am Werkzeug durch eine translatorische Bewegung des Außenschafts relativ zum Werkzeug herstellbar und die mechanischen Verbindung der Werkzeug-Kupplungseinrichtung an der Übertragungseinrichtung mit der Übertragungs-Kupplungseinrichtung am Werkzeug durch eine rotatorische Bewegung der Übertragungseinrichtung relativ zum Werkzeug herstellbar. Oder umgekehrt sind die mechanischen Verbindung der Werkzeug-Kupplungseinrichtung am Außenschaft mit der Außenschaft-Kupplungseinrichtung am Werkzeug durch eine rotatorische Bewegung des Außenschafts relativ zum Werkzeug herstellbar und die mechanischen Verbindung der Werkzeug-Kupplungseinrichtung an der Übertragungseinrichtung mit der Übertragungs-Kupplungseinrichtung am Werkzeug durch eine translatorische Bewegung der Übertragungseinrichtung relativ zum Werkzeug herstellbar. Die Rotationsachse der rotatorischen Bewegung ist dabei insbesondere parallel zur Bewegungsrichtung der translatorischen Bewegung.

Bei einem medizinischen Instrument mit den beschriebenen Merkmalen können somit zwei mechanische Verbindungen, nämlich die des Werkzeugs mit dem Außenschaft einerseits und mit der Übertragungseinrichtung andererseits, durch eine einzige Einrichtung verriegelt werden. Diese Verriegelung kann überdies an den proximalen Enden von Außenschaft und Übertragungseinrichtung erfolgen, so dass an deren distalen Enden Bauraum eingespart werden kann.

Bei einem medizinischen Instrument, wie es hier beschrieben ist, sind insbesondere die mechanische Verbindung der Werkzeug-Kupplungseinrichtung am Außenschaft mit der Außenschaft-Kupplungseinrichtung am Werkzeug durch eine Rotation des Außenschafts in einer ersten Richtung relativ zum Werkzeug herstellbar und die mechanische Verbindung der Werkzeug-Kupplungseinrichtung an der Übertragungseinrichtung mit der Übertragungs-Kupplungseinrichtung am Werkzeug durch eine Rotation der Übertragungseinrichtung in einer zweiten Richtung relativ zum Werkzeug herstellbar.

Bei einem medizinischen Instrument, wie es hier beschrieben ist, umfassen insbesondere zumindest entweder die Außenschaft-Kupplungseinrichtung am Werkzeug und die Werkzeug-Kupplungseinrichtung am Außenschaft oder die Übertragungs-Kupplungseinrichtung am Werkzeug und die Werkzeug-Kupplungseinrichtung an der Übertragungseinrichtung Schraubgewinde.

Insbesondere umfassen die Außenschaft-Kupplungseinrichtung am Werkzeug und die Werkzeug-Kupplungseinrichtung am Außenschaft jeweils Links-Gewinde und die Übertragungs-Kupplungseinrichtung am Werkzeug und die Werkzeug-Kupplungseinrichtung an der Übertragungseinrichtung jeweils Rechts-Gewinde oder umgekehrt.

Bei einem medizinischen Instrument, wie es hier beschrieben ist, umfassen insbesondere zumindest entweder die Außenschaft-Kupplungseinrichtung am Werkzeug und die Werkzeug-Kupplungseinrichtung am Außenschaft oder die Übertragungs-Kupplungseinrichtung am Werkzeug und die Werkzeug-Kupplungseinrichtung an der Übertragungseinrichtung Bajonettkupplungen.

Insbesondere Bajonettkupplungen werden bereits an herkömmlichen zerlegbaren medizinischen Instrumenten verwendet und sind deshalb konstruktiv und fertigungstechnisch gut beherrscht. Gegenüber der herkömmlichen Verriegelung jeder einzelnen Bajonettkupplung durch einen Riegel an der Bajonettkupplung selbst (oft auch als Pendelplatte bezeichnet) oder durch einen Riegel am proximalen Ende des medizinischen Instruments (oft auch als "Halbschalen"-Lösung bezeichnet) ermöglicht ein medizinisches Instrument mit den hier beschriebenen Merkmalen eine einzige Verriegelungseinrichtung für zwei Bajonettkupplungen und damit ein deutliches Ersparnis an Bauraum, konstruktivem und fertigungstechnischem Aufwand.

Bei einem medizinischen Instrument, wie es hier beschrieben ist, umfasst die Übertragungseinrichtung insbesondere einen Innenschaft.

Ein medizinisches Instrument, wie es hier beschrieben ist, umfasst insbesondere ferner eine Übertragungsstange zum Übertragen zumindest entweder einer Kraft oder eines Drehmoments, wobei die Übertragungsstange zum Steuern einer ersten Funktion und der Innenschaft zum Steuern einer zweiten Funktion ausgebildet sind.

Sowohl für den Innenschaft als auch für die Übertragungsstange gilt, dass sie im Falle eines gekrümmten oder krümmbaren bzw. biegeelastischen Außenschafts insbesondere zumindest abschnittsweise biegeelastisch ausgebildet sind. Die Übertragungsstange ist insbesondere im Inneren bzw. im Lumen des rohrförmig ausgebildeten Innenschafts angeordnet. Der Innenschaft ist insbesondere im ringförmigen Zwischenraum zwischen dem Außenschaft und der Übertragungsstange angeordnet.

Die mittels der Übertragungsstange zu steuernde erste Funktion ist beispielsweise eine Greiffunktion. Die mittels des Innenschafts zu steuernde zweite Funktion ist insbesondere eine Schneidefunktion.

Bei einem medizinischen Instrument, wie es hier beschrieben ist, sind insbesondere die Übertragungsstange mit einem schwenkbaren Maulteil des Werkzeugs gekoppelt und der Innenschaft für eine mechanische Verbindung mit einer Schneideeinrichtung des Werkzeugs ausgebildet.

Insbesondere ist das distale Ende der Übertragungsstange derart mit einem oder mehreren schwenkbaren Maulteilen gekoppelt, dass eine Bewegung (insbesondere eine translatorische Bewegung parallel zur Längsachse des Außenschafts) der Übertragungsstange ein Schwenken des oder der schwenkbaren Maulteile um je eine zur Längsachse des Außenschafts senkrechte Schwenkachse bewirkt. Die Schneideeinrichtung ist insbesondere ein Messer oder Skalpell, das im Werkzeug in Richtung parallel zur Längsachse des Außenschafts verschiebbar ist. Die Maulteile sind insbesondere so ausgebildet, dass auch in der geschlossenen bzw. greifenden Konfiguration der Maulteile zwischen diesen ein Kanal bzw. ein Lumen verbleibt, in dem die Schneideeinrichtung bewegbar ist, um mittels der Maulteile gegriffenes Gewebe zu durchtrennen. Dabei ist die Schneideeinrichtung insbesondere so gelagert, dass sie nicht um die Längsachse des Werkzeugs bzw. des Außenschafts rotierbar ist. Insbesondere ist die Schneideeinrichtung in einer Nut in der Übertragungsstange und im Werkzeug angeordnet, wobei die Nut einen zu der Schneideeinrichtung korrespondierenden, im Wesentlichen rechteckigen Querschnitt aufweist.

Alternativ kann das medizinische Instrument zwei Übertragungsstangen zum Steuern von zwei beliebigen voneinander verschiedenen Funktionen des Werkzeugs aufweisen, wobei zumindest eine Übertragungsstange und der Außenschaft durch die oben beschriebenen, einander entgegengerichteten Bewegungen mit dem Werkzeug koppelbar sind.

Bei einem medizinischen Instrument, wie es hier beschrieben ist, umfasst die Übertragungs-Kupplungseinrichtung am Werkzeug insbesondere einen Vorsprung, der in einer Richtung senkrecht zur vorgesehenen Bewegungsrichtung der Schneideeinrichtung vorsteht, wobei die Werkzeug-Kupplungseinrichtung an der Übertragungseinrichtung einen L-förmigen Schlitz oder eine L-förmige Nut zur Aufnahme des Vorsprungs umfasst.

Der Vorsprung und der L-förmige Schlitz bzw. die L-förmige Nut bilden zueinander korrespondierende Bajonett-Kupplungseinrichtungen, wobei der Vorsprung an der Schneideeinrichtung die Funktion einer Knagge bzw. Klaue aufweist. Der Vorsprung ist insbesondere am proximalen Ende oder nahe dem proximalen Ende der Schneideeinrichtung angeordnet. Wenn die Schneideeinrichtung, wie oben beschrieben, in einer Nut in einer Übertragungsstange angeordnet ist, ist der Vorsprung insbesondere so ausgebildet, dass er über die Ränder der Nut vorsteht, wobei andere Bereiche der Schneideeinrichtung vollständig in der Nut angeordnet sind. Die bajonettartige Kupplung der Schneideeinrichtung mit der Übertragungseinrichtung ist mit einem besonders geringen Bauraumbedarf realisierbar.

Bei einem medizinischen Instrument, wie es hier beschrieben ist, weist das Werkzeug insbesondere zwei voneinander elektrisch isolierte Maulteile auf, wobei ein erstes der zwei voneinander elektrisch isolierten Maulteile mit einer Übertragungsstange elektrisch leitfähig verbunden ist, und wobei ein zweites der zwei voneinander elektrisch isolierten Maulteile mit dem Außenschaft elektrisch leitfähig verbunden ist.

Damit ist das medizinische Instrument insbesondere in der Elektrochirurgie einsetzbar. In der Elektrochirurgie bzw. bei elektrochirurgischen Maßnahmen wird durch Stromfluss und aufgrund des elektrischen Widerstands von Gewebe (Joulesche) Wärme im Gewebe erzeugt. Durch Form und Anordnung von dabei verwendeten Elektroden, insbesondere zweier oder mehrerer Maulteile, wird der Stromfluss möglichst genau lokalisiert. Durch die entstehende Wärme wird das stromdurchflossene Gewebe verödet bzw. zerstört. Dadurch können Gewebe beispielsweise verklebt oder verschlossen und Blutungen gestillt werden.

In der Regel werden in der Elektrochirurgie hochfrequente Wechselströme verwendet, um eine Stimulation von Nerven und andere unerwünschte Nebenwirkungen zu vermeiden. Die Begriffe "Elektrochirurgie" und "HF-Chirurgie" werden deshalb oft synonym verwendet. Ein weiterer, oft synonym verwendeter Begriff ist der der Elektrokauterisation.

Ein medizinisches Instrument mit den hier beschriebenen Merkmalen kann ein Greifen von Gefäßen oder anderem Gewebe, eine Elektrokauterisation des gegriffenen Gewebes und eine nachfolgende Durchtrennung mittels der Schneideeinrichtung ermöglichen. Diese Funktionen sind aufgrund der beschriebenen mechanischen Verbindung des Werkzeugs mit dem Außenschaft und der Übertragungseinrichtung trotz einer Zerlegbarkeit bei einem vergleichsweise geringen Querschnitt des Schafts und des Werkzeugs realisierbar.

Bei einem medizinischen Instrument, wie es hier beschrieben ist, weist der Innenschaft insbesondere einen distalen Abschnitt und einen vom distalen Abschnitt elektrisch isolierten proximalen Abschnitt auf.

Insbesondere ist der Innenschaft nicht Bestandteil des Stromkreises. Im Falle einer elektrischen Isolation eines distalen Abschnitts von einem proximalen Abschnitt des Innenschafts können der distale Abschnitt des Innenschafts mit der Übertragungsstange und gleichzeitig der proximale Abschnitt des Innenschafts mit dem Außenschaft elektrisch leitfähig verbunden sein, ohne dadurch eine elektrisch leitfähige Verbindung zwischen Übertragungsstange und Außenschaft zu schaffen. Dadurch können sowohl der distale Abschnitt des Innenschafts mit einer Bajonett-Kupplungseinrichtung oder einer anderen Werkzeug-Kupplungseinrichtung zur mechanischen Verbindung mit dem Werkzeug (insbesondere der oben beschriebenen Schneideeinrichtung) als auch der proximale Abschnitt des Innenschafts aus Metall gebildet sein und damit eine große Festigkeit, eine große Härte und günstige Verschleißeigenschaften aufweisen. Am proximalen Ende des Innenschafts kann dies insbesondere hinsichtlich der unten beschriebenen, zu einem Riegel am Außenschaft korrespondierenden Ausnehmung vorteilhaft sein.

Ein medizinisches Instrument, wie es hier beschrieben ist, umfasst insbesondere ferner eine Handhabungseinrichtung zur lösbaren mechanischen Verbindung mit den proximalen Enden des Außenschafts und der Übertragungseinrichtung, wobei die Handhabungseinrichtung, der Außenschaft und die Übertragungseinrichtung so ausgebildet sind, dass der Außenschaft und die Übertragungseinrichtung nicht parallel zur ersten und zur zweiten Richtung relativ zueinander bewegbar sind, wenn sie mit der Handhabungseinrichtung in einer vorgesehenen Weise mechanisch verbunden sind.

Die Hemmung der Bewegung von Außenschaft und Übertragungseinrichtung relativ zueinander parallel zur ersten und zur zweiten Richtung wird nachfolgend auch als Verriegelung des Außenschafts und der Übertragungseinrichtung miteinander bezeichnet. Diese Verriegelung schließt nicht aus, dass Außenschaft und Übertragungseinrichtung in einer Richtung senkrecht zur ersten und zur zweiten Richtung relativ zueinander bewegbar sind. Ebenso wenig schließt die Verriegelung von Außenschaft und Übertragungseinrichtung miteinander aus, dass diese gemeinsam in einer Richtung parallel und/oder senkrecht zur ersten und zur zweiten Richtung bewegbar sind.

Die Verriegelung des Außenschafts und der Übertragungseinrichtung miteinander bewirkt aufgrund der zum Verbinden und damit auch zum Trennen der mechanischen Verbindungen des Werkzeugs mit dem Außenschaft einerseits und mit der Übertragungseinrichtung andererseits erforderlichen entgegengesetzten Bewegungen eine Verriegelung der mechanischen Verbindungen zwischen dem Werkzeug einerseits und dem Außenschaft und der Übertragungseinrichtung andererseits. Somit kann allein eine mechanische Verbindung der proximalen Enden des Außenschafts und der Übertragungseinrichtung mit der Handhabungseinrichtung in der vorgesehenen Weise, insbesondere eine Verriegelung des Außenschafts an der Handhabungseinrichtung, gleichzeitig eine Verriegelung der mechanischen Verbindungen zwischen dem Werkzeug einerseits und dem Außenschaft und der Übertragungseinrichtung andererseits bewirken.

Bei einem medizinischen Instrument, wie es hier beschrieben ist, umfasst der Außenschaft insbesondere einen Riegel, der zwischen einer entriegelnden Position und einer verriegelnden Position bewegbar und ausgebildet ist, um in der verriegelnden Position in eine korrespondierende Ausnehmung an der Übertragungseinrichtung einzugreifen, wobei die Handhabungseinrichtung ausgebildet ist, um den Riegel in der verriegelnden Position zu halten, wenn der Außenschaft mit der Handhabungseinrichtung mechanisch verbunden ist.

Der Riegel am Außenschaft wird auch als Halbschale bezeichnet. Der Riegel ist insbesondere in radialer Richtung zwischen der radial außenliegenden entriegelnden Position und der radial innenliegenden verriegelnden Position bewegbar. Der Riegel ist insbesondere ausgebildet, um in der entriegelnden Position über die Außenkontur des Außenschafts überzustehen und in der verriegelnden Position mit der Außenkontur des Außenschafts im Wesentlichen bündig abzuschließen.

Ein oder mehrere O-Ringe, Blattfedern oder andere elastische Elemente können vorgesehen und ausgebildet sein, um auf den Riegel eine Kraft in Richtung zu der verriegelnden Position auszuüben. Wenn der Außenschaft in der vorgesehenen und vorbestimmten Weise mit der Handhabungseinrichtung verbunden ist, ist er beispielsweise in eine Ausnehmung mit korrespondierendem Querschnitt aufgenommen. Dabei liegt insbesondere die innere Oberfläche der Ausnehmung an einer radial außenliegenden Oberfläche des Riegels an und hält ihn so in der verriegelnden Position.

Wenn die erste Richtung und die zweite Richtung jeweils parallel oder im Wesentlichen parallel zur umfänglichen Richtung sind (insbesondere im Falle von Schraubgewinde- oder Bajonettkupplungen), erstreckt sich die Ausnehmung insbesondere in Form einer Nut oder einer Abflachung an der Übertragungseinrichtung in axialer Richtung und damit senkrecht zur ersten und zur zweiten Richtung. Die Ausnehmung erstreckt sich insbesondere so weit in axialer Richtung, dass Außenschaft und Übertragungseinrichtung relativ zueinander in axialer Richtung innerhalb eines vorbestimmten Bereichs bewegbar sind.

Wenn die erste Richtung und die zweite Richtung jeweils parallel zur axialen Richtung sind, ist die Ausnehmung insbesondere als sich in umfänglicher Richtung erstreckende Nut an der Übertragungseinrichtung ausgebildet. Dadurch können Außenschaft und Übertragungseinrichtung relativ zueinander rotiert werden.

Bei einem medizinischen Instrument, wie es hier beschrieben ist, sind Außenschaft und Innenschaft insbesondere gemeinsam in Richtung parallel zur ersten und zur zweiten Richtung relativ zur Handhabungseinrichtung bewegbar, wenn sie mit der Handhabungseinrichtung mechanisch verbunden sind.

Dies ermöglicht insbesondere im Falle von Bajonettkupplungen zwischen dem Werkzeug einerseits und dem Außenschaft und der Übertragungseinrichtung andererseits eine Rotation des Werkzeugs um seine Längsachse bzw. um die Längsachse des Außenschafts.

Bei einem Verfahren zum Zusammensetzen eines Werkzeugs, einer Übertragungseinrichtung zum Übertragen zumindest entweder einer Kraft oder eines Drehmoments und eines Außenschafts zu einem medizinischen Instrument werden die Übertragungseinrichtung mit dem Werkzeug mechanisch verbunden, wobei das Verbinden ein Bewegen der Übertragungseinrichtung in einer ersten Richtung relativ zum Werkzeug umfasst, und der Außenschaft mit dem Werkzeug mechanisch verbunden, wobei das Verbinden ein Bewegen des Außenschafts in einer zweiten Richtung relativ zum Werkzeug umfasst, und wobei die erste Richtung entgegengesetzt zur zweiten Richtung ist.

Das Werkzeug, die Übertragungseinrichtung, der Außenschaft und das aus diesen gebildete medizinische Instrument weisen insbesondere die oben beschriebenen Merkmale auf. Das Verfahren zum Zusammensetzen kann der Vorbereitung der Verwendung des medizinischen Instruments dienen, insbesondere auch der Vorbereitung der Verwendung im Rahmen eines diagnostischen, chirurgischen oder therapeutischen Verfahrens. Das Verfahren zum Zusammensetzen stellt aber selbst kein diagnostisches, chirurgisches oder therapeutisches Verfahren dar.

Bei einem Verfahren, wie es hier beschrieben ist, werden insbesondere ferner das proximale Ende des Außenschafts und das proximale Ende der Übertragungseinrichtung derart gekoppelt, dass der Außenschaft und die Übertragungseinrichtung nicht parallel zur ersten und zur zweiten Richtung relativ zueinander bewegbar sind.

Das proximale Ende des Außenschafts und das proximale Ende der Übertragungseinrichtung werden insbesondere nach dem mechanischen Verbinden des Innenschafts und des Außenschafts mit dem Werkzeug gekoppelt. Das Koppeln der proximalen Enden von Außenschaft und Übertragungseinrichtung schließt insbesondere nicht aus, dass Außenschaft und Übertragungseinrichtung gemeinsam parallel zur ersten und zur zweiten Richtung bewegbar sind. Das Koppeln der proximalen Enden von Außenschaft und Übertragungseinrichtung schließt ferner insbesondere nicht aus, dass Außenschaft und Übertragungseinrichtung relativ zueinander senkrecht zur ersten und zur zweiten Richtung bewegbar sind.

Bei einem Verfahren, wie es hier beschrieben ist, wird ferner insbesondere das proximale Ende des Außenschafts mit einer Handhabungseinrichtung mechanisch verbunden, wobei während des Verbindens des proximalen Endes des Außenschafts mit der Handhabungseinrichtung die proximalen Enden von Außenschaft und Übertragungseinrichtung miteinander gekoppelt werden.

Das proximale Ende des Außenschafts wird insbesondere nach dem mechanischen Verbinden des Innenschafts und des Außenschafts mit dem Werkzeug mit der Handhabungseinrichtung verbunden.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine schematische axonometrische Darstellung einer Greifeinrichtung für ein medizinisches Instrument;
- Figur 3: eine schematische axonometrische Darstellung einer Schneideeinrichtung;
- Figur 4: eine weitere schematische axonometrische Darstellung der Einrichtungen aus den Figur 2 und 3;
- Figur 5: eine weitere schematische axonometrische Darstellung der Einrichtungen aus den Figuren 2 bis 4;
- Figur 6: eine schematische axonometrische Darstellung eines proximalen Endes eines Innenschafts;
- Figur 7: eine schematische Schnittdarstellung eines Schafts;
- Figur 8: eine weitere schematische Schnittdarstellung des Schafts aus Figur7;
- Figur 9: eine schematische Schnittdarstellung eines weiteren Werkzeugs für ein medizinisches Instrument;
- Figur 10: eine weitere schematische Schnittdarstellung des Werkzeugs aus Figur 9;
- Figur 11: eine schematische Schnittdarstellung eines Außenschafts und einer Übertragungseinrichtung;
- Figur 12: eine weitere schematische Schnittdarstellung des Außenschafts und der Übertragungseinrichtung aus Figur 11;
- Figur 13: ein schematisches Flussdiagramm eines Verfahrens zum Zusammensetzen eines medizinischen Instruments.

### Beschreibung der Ausführungsformen

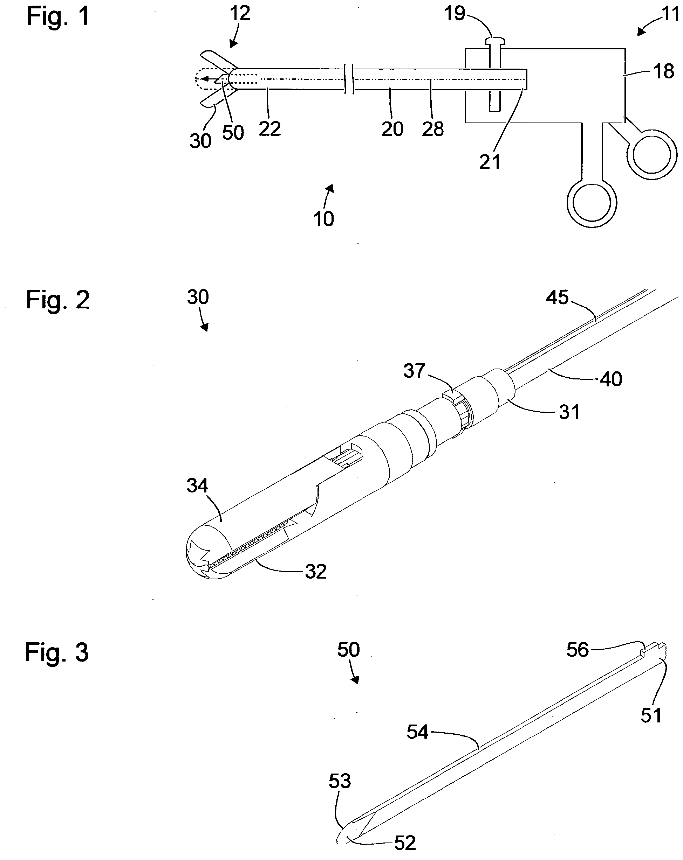

Figur 1 zeigt eine schematische Darstellung eines medizinischen Instruments 10 mit einem proximalen Ende 11 und einem distalen Ende 12. Am proximalen Ende 11 weist das medizinische Instrument 10 eine Handhabungseinrichtung 18 auf, die dazu ausgebildet ist, von medizinischem Personal mit einer Hand gefasst zu werden und manuell erzeugte Kräfte und Momente aufzunehmen. Dazu weist die Handhabungseinrichtung 18 insbesondere mehrere Griffteile auf, die zumindest teilweise relativ zueinander bewegbar sind.

Ein Schaft 20 erstreckt sich vom proximalen Ende 11 bzw. der Handhabungseinrichtung 18 bis zum distalen Ende 12 des medizinischen Instruments 10. Der Schaft 20 weist ein proximales Ende 21 und ein distales Ende 22 auf. Das proximale Ende 21 des Schafts 20 ist mit der Handhabungseinrichtung 18 mechanisch verbunden, insbesondere in einer Ausnehmung mit einer zum proximalen Ende 21 des Schafts 20 korrespondierenden Gestalt angeordnet und dort mittels einer Verriegelungseinrichtung 19 formschlüssig verriegelt.

Der Schaft 20 weist eine Längsachse 28 auf. Im Falle einer kreiszylindrischen Gestalt des Schafts 20 ist die Längsachse 28 insbesondere die Symmetrieachse der Mantelfläche des Schafts 20. Auch der unten beschriebene innere Aufbau des Schafts 20 kann rotationssymmetrisch zur Längsachse 28 sein. Der Schaft 20 kann gerade oder - abweichend von der Darstellung in Figur 1 - gekrümmt, starr oder flexibel sein. Wenn der Schaft 20 zumindest abschnittsweise gekrümmt oder flexibel ist, ist mit der Längsachse nachfolgend die Längsachse des Schafts 20 an seinem proximalen Ende 21 oder an seinem distalen Ende 22 gemeint. Der Schaft 20 ist insbesondere auch im in der Handhabungseinrichtung 18 verriegelten Zustand um seine Längsachse 28 rotierbar.

Das distale Ende 22 des Schafts 20 ist mit einem Werkzeug verbunden, das eine Greifeinrichtung 30 und eine Schneideeinrichtung 50 umfasst. Die Greifeinrichtung 30 weist insbesondere zwei Greifbacken auf, von denen mindestens eine um eine Schwenkachse senkrecht zur Zeichenebene der Figur 1 schwenkbar ist. Bei dem in Figur 1 gezeigten Beispiel sind beide Greifbacken zwischen offenen Positionen, die in Figur 1 mit durchgezogenen Linien dargestellt sind, und geschlossenen Positionen, die in Figur 1 mit gestrichelten Linien dargestellt sind, schwenkbar. Die Schneideeinrichtung 50 ist, wie in Figur 1 durch einen Pfeil angedeutet, in Richtung parallel zur Längsachse 28 bewegbar, und zwar sowohl wenn die Maulteile der Greifeinrichtung ihre offenen als auch wenn sie ihre geschlossenen Positionen einnehmen.

Nachfolgend sind Ausführungsbeispiele des Werkzeugs 30, 50 und seiner lösbaren mechanischen Verbindung mit einem Außenschaft, einem Innenschaft und einer Übertragungsstange dargestellt. Die nachfolgend dargestellten Werkzeuge, Außenschäfte, Innenschäfte und Übertragungsstangen können zur Bildung eines medizinischen Instruments mit den anhand der Figur 1 dargestellten Merkmalen und/oder mit anderen Merkmalen ausgebildet sein und verwendet werden.

Figur 2 zeigt eine schematische axonometrische Darstellung einer Greifeinrichtung 30, die vorgesehen und ausgebildet ist zur Bildung eines medizinischen Instruments, wie es oben anhand der Figur 1 dargestellt ist. Die Greifeinrichtung 30 weist ein proximales Ende 31 und zwei Maulteile 32, 34, die das distale Ende der Greifeinrichtung 30 bilden, auf. Im Gegensatz zu der in Figur 1 angedeuteten Greifeinrichtung umfasst die in Figur 2 dargestellte Greifeinrichtung 30 ein feststehendes Maulteil 32 und ein schwenkbares Maulteil 34. Nahe dem proximalen Ende 31 weist die Greifeinrichtung 30 zwei symmetrisch angeordnete Bajonettklauen bzw. Knaggen 37 auf, von denen eine an einer vom Betrachter abgewandten Seite angeordnet und deshalb weitgehend verdeckt ist.

Die Greifeinrichtung 30 ist mit einer Übertragungsstange 40 mechanisch verbunden. Die Übertragungsstange 40 ist relativ zur Greifeinrichtung 30, insbesondere relativ zum proximalen Ende 31 und zum feststehenden Maulteil 32 in axialer Richtung, d.h. parallel zur Längsachse der Übertragungsstange 40 und zur Längsachse 28 des Schafts 20 (vgl. Figur 1) innerhalb eines vorbestimmten Intervalls bewegbar. Das innerhalb der Greifeinrichtung 30 angeordnete und deshalb in Figur 2 nicht sichtbare distale Ende der Übertragungsstange 40 ist mit dem schwenkbaren Maulteil 34 derart gekoppelt, dass eine axiale Bewegung der Übertragungsstange 40 mit einer Schwenkbewegung des schwenkbaren Maulteils 34 einhergeht. In der Übertragungsstange 40 ist eine Nut 45 vorgesehen, die insbesondere einen schmalen und tiefen rechteckigen Querschnitt aufweist. Die Nut 45 in der Übertragungsstange 40 ist an deren in Figur 2 nicht sichtbaren distalen Ende durch einen Kanal entsprechenden Querschnitts fortgesetzt, der sich zwischen den Maulteilen 32, 34 bis fast zu deren distalen Enden erstreckt.

Teile der Greifeinrichtung 30, insbesondere die Knaggen 37 sowie die Übertragungsstange 40 sind aus Edelstahl oder einem anderen Metall gefertigt. Die Knaggen 37 und die Übertragungsstange 40 sind voneinander elektrisch isoliert. Die Maulteile 32, 34 weisen metallische und damit elektrisch leitfähige Greifflächen auf, die voneinander elektrisch isoliert sind, wenn sie nicht, wie in Figur 2 gezeigt, aneinander anliegen. Die Knaggen 37 und die Übertragungsstange 40 sind jeweils mit der Greiffläche eines Maulteils 32, 34 elektrisch leitfähig verbunden. Insbesondere sind die Knaggen 37 mit der Greiffläche des feststehenden Maulteils 32 und die Übertragungsstange 40 mit der Greiffläche des schwenkbaren Maulteils 34 elektrisch leitfähig verbunden.

Figur 3 zeigt eine schematische axonometrische Darstellung einer Schneideeinrichtung 50 mit einem proximalen Ende 51 und einem distalen Ende 52. Am distalen Ende 52 weist die Schneideeinrichtung 50 eine Schneide 53 auf. Am proximalen Ende weist die Schneideeinrichtung 50 einen Vorsprung 56 auf. Zwischen dem proximalen Ende 51 und dem distalen Ende 52 umfasst die Schneideeinrichtung einen stabförmigen Bereich 54, der im Wesentlichen die Gestalt einer streifenförmigen Platte bzw. eines Stabs mit rechteckigem Querschnitt aufweist.

Zwischen dem Vorsprung 56 am proximalen Ende 51 und der Schneide 53 am distalen Ende 52 entspricht der Querschnitt der Schneideeinrichtung 50 im Wesentlichen dem Querschnitt der Nut 45 in der Übertragungsstange 40 (vgl. Figur 2), so dass die Schneideeinrichtung 50 von dem Vorsprung 56 abgesehen, vollständig von der Nut 45 in der Übertragungsstange 40 aufgenommen und in dieser spiel- und reibungsarm geführt und in Längsrichtung der Übertragungsstange 40 und der Schneideeinrichtung 50 verschiebbar sein kann. Der Vorsprung 56 ist vorgesehen, um aus der Nut 45 in der Übertragungsstange 40 herauszuragen.

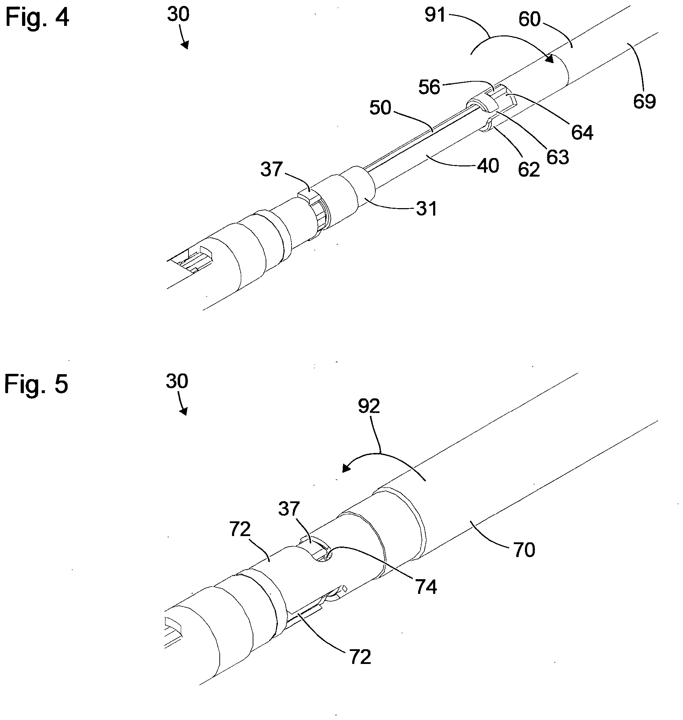

Figur 4 zeigt eine weitere schematische axonometrische Darstellung der Greifeinrichtung aus Figur 2 und der Schneideeinrichtung 50 aus Figur 3. Bei der Darstellung in Figur4 ist die Schneideeinrichtung 50 in der Nut 45 in der Übertragungsstange 40 (vgl. Figur 2) angeordnet. Das distale Ende 52 der Schneideeinrichtung 50 (vgl. Figur 3) ist dabei in der Greifeinrichtung und insbesondere zwischen den Maulteilen 32, 34 angeordnet. Der Vorsprung 56 ragt aus der Nut 45 heraus.

In Figur 4 ist ferner ein Innenschaft 60 gezeigt, der im Wesentlichen eine rohrförmige bzw. kreiszylindermantelförmige Gestalt aufweist. Der Innenschaft 60 weist an seinem distalen Ende 62 einen L-förmigen Schlitz mit einem axialen bzw. sich in axialer Richtung erstreckenden Abschnitt 63 und einem umfänglichen bzw. in umfänglicher Richtung sich erstreckenden Abschnitt 64 auf. Die in umfänglicher Richtung zu messende Breite des axialen Abschnitts 63 und die in axialer Richtung zu messende Breite des umfänglichen Abschnitts 64 des L-förmigen Schlitzes sind an die Abmessungen des Vorsprungs 56 an der Schneideeinrichtung 50 angepasst.

Nach einem Einführen der Übertragungsstange 40 in den Innenschaft 60 kann der Vorsprung 56 durch eine Relativbewegung in axialer Richtung durch den axialen Abschnitt 63 bis in den umfänglichen Abschnitt 64 eingeführt werden. Wenn der Vorsprung 56 an der Schneideeinrichtung 50 sich im umfänglichen Abschnitt 64 des L-förmigen Schlitzes am distalen Ende 62 des Innenschafts 60 befindet, kann der Innenschaft 60 relativ zu der Greifeinrichtung 30, der Übertragungsstange 40 und der Schneideeinrichtung 50 in einer ersten Richtung 91 bis zu der in Figur 4 gezeigten Konfiguration rotiert werden.

In der in Figur 4 gezeigten relativen Positionierung von Schneideeinrichtung 50 und Innenschaft 60 sind Schneideeinrichtung 50 und Innenschaft 60 hinsichtlich axialer Bewegungen miteinander (von Spiel abgesehen) starr gekoppelt. Eine axiale Bewegung des Innenschafts 60 geht deshalb mit einer entsprechenden axialen Bewegung der Schneideeinrichtung 50 einher. Somit kann mittels des Innenschafts 60 eine Bewegung der Schneide 53 am distalen Ende 52 der Schneideeinrichtung 50 (vgl. Figur 3) in dem erwähnten, in den Figuren nicht sichtbaren Kanal zwischen den Maulteilen 32, 34 bewirkt werden, um beispielsweise von den Maulteilen 32, 34 gegriffenes Gewebe nach einer Elektrokauterisation zu durchtrennen.

Der Innenschaft 60 weist einen Isoliermantel 69 auf, dessen distaler Rand nahe dem L-förmigen Schlitz 63, 64 liegt, und der sich bis nahe des proximalen Endes des Innenschafts 60 erstrecken kann.

Figur 5 zeigt eine weitere schematische axonometrische Darstellung der Greifeinrichtung 30 aus den Figuren 2 und 4. Die Darstellung in Figur 5 unterscheidet sich von der Darstellung in den Figuren 2 und 4 dadurch, dass ein Außenschaft 70 mit der Greifeinrichtung 30 gekoppelt ist. Der Außenschaft 70 weist an seinem distalen Ende 72 zwei symmetrisch angeordnete L-förmige Schlitze mit je einem axialen Abschnitt 73 und einen umfänglichen Abschnitt 74 auf. Einer der L-förmigen Schlitze ist an einer vom Betrachter abgewandten Seite des Außenschafts 70 angeordnet und deshalb in Figur 5 nicht sichtbar.

Die in umfänglicher Richtung zu messende Breite der axialen Abschnitte 73 und die in axialer Richtung zu messende Breite der umfänglichen Abschnitte 74 der L-förmigen Schlitze sind an die Abmessungen der Knaggen 37 an der Greifeinrichtung 30 angepasst. Durch eine Bewegung des Außenschafts 70 in axialer Richtung und eine nachfolgende Rotation relativ zur Greifeinrichtung 30 können die Knaggen 37 durch die axialen Abschnitte 73 in die umfänglichen Abschnitte 74 eingeführt werden bis zu der in Figur 5 gezeigten Konfiguration. Bei der in Figur 5 gezeigten Konfiguration bzw. Anordnung der Knaggen 37 in den umfänglichen Abschnitten 74 der L-förmigen Schlitze am distalen Ende 72 des Außenschafts 70 sind der Außenschaft 70 und die Greifeinrichtung 30 hinsichtlich axialer Kräfte und Bewegungen miteinander (von Spiel abgesehen) starr gekoppelt.

Im Vergleich der Figuren 4 und 5 ist erkennbar, dass die umfänglichen Abschnitte 64, 74 der L-förmigen Schlitze an den distalen Enden 62, 72 des Innenschafts 60 einerseits und des Außenschafts 70 andererseits sich von den axialen Abschnitten 63, 73 aus in entgegengesetzten Richtungen erstrecken. Entsprechend ist die Richtung 92, in der der Außenschaft 70 relativ zur Greifeinrichtung 30 zu rotieren ist, um die in Figur 5 gezeigte gekoppelte Konfiguration zu erreichen, entgegengesetzt zu der Richtung 91, in der der Innenschaft 60 relativ zur Greifeinrichtung 30 und zur Schneideeinrichtung 50 zu rotieren ist, um die in Figur 4 gezeigte gekoppelte Konfiguration zu erreichen.

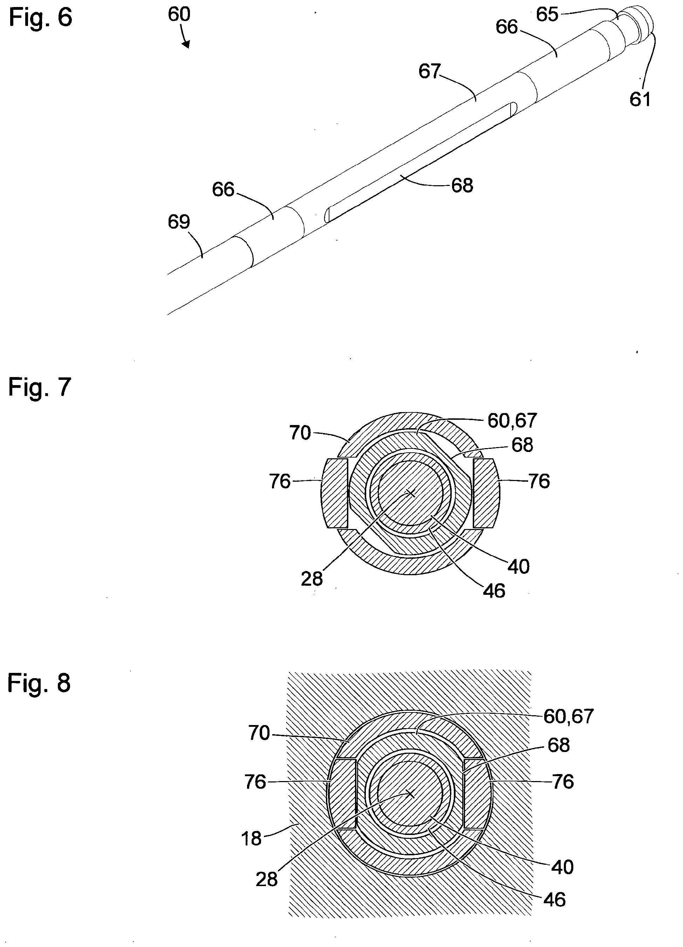

Figur 6 zeigt eine schematische axonometrische Darstellung eines proximalen Bereichs des Innenschafts 60 aus Figur 4. Der Innenschaft 60 weist an seinem proximalen Ende 61 ein metallisches Kupplungsbauteil 65 zur lösbaren, unmittelbaren oder mittelbaren mechanischen Kopplung mit einem bewegbaren Griffteil einer Handhabungseinrichtung auf. Das Kupplungsbauteil 65 ist durch ein Isolierbauteil 66 mit einem metallischen Bauteil 67 mechanisch starr verbunden und gleichzeitig von diesem elektrisch isoliert. Das metallische Bauteil 67 weist zwei symmetrisch zueinander angeordnete Abflachungen 68 auf, von denen in Figur 6 nur eine dem Betrachter zugewandt und damit sichtbar ist. Das metallische Bauteil 67 ist durch ein weiteres Isolierbauteil 66 mit einem Metallrohr, das von dem Isoliermantel 69 umgeben ist und sich bis zu dem in Figur 4 gezeigten distalen Ende 62 erstreckt, mechanisch verbunden und von diesem elektrisch isoliert.

Figur 7 zeigt eine schematische Darstellung eines Schnitts durch die Übertragungsstange 40, den Innenschaft 60 und den Außenschaft 70 im Bereich des metallischen Bauteils 67 des Innenschafts 60 (vgl. Figur 6). Die dargestellte Schnittebene ist senkrecht zur Längsachse 28 (vgl. Figur 1). In der dargestellten Schnittebene weist die Übertragungsstange 40 einen Isoliermantel 46 auf, der die Übertragungsstange 40 vom Innenschaft 60 elektrisch isoliert. Der Außenschaft 70 weist zwei einander gegenüberliegende Öffnungen auf, in denen Riegel 76 radial verschiebbar angeordnet sind. Die Riegel 76 werden beispielsweise durch einen oder mehrere O-Ringe, Blattfedern oder andere elastische Einrichtungen nach radial innen gedrückt und/oder durch andere in Figur 7 nicht gezeigte Einrichtungen in den Ausnehmungen im Außenschaft 70 gehalten.

Figur 8 zeigt eine weitere schematische Schnittdarstellung der Übertragungseinrichtung 40, des Innenschafts 60 und des Außenschafts 70. Die in Figur 8 gezeigte Schnittebene entspricht der Schnittebene der Figur 7. Die Darstellung in Figur 8 unterscheidet sich von der Darstellung in Figur 7 insbesondere dadurch, dass der Innenschaft 60 um die Längsachse 28 so weit rotiert ist, dass die Riegel 76 nach radial innen verschoben sein können und flächig an den Abflachungen 68 am metallischen Bauteil 67 des Innenschafts 60 anliegen. Dabei schließen die Riegel 76 mit der kreisförmigen äußeren Kontur des Außenschafts 70 bündig ab. In dieser Konfiguration kann der Außenschaft 70 mit den Riegeln 76, dem Innenschaft 60 und der Übertragungsstange 40 in eine korrespondierende Ausnehmung in der Handhabungseinrichtung 18 (vgl. Figur 1) eingesetzt werden, um die in Figur 8 gezeigte Konfiguration zu erreichen.

Bei der in Figur 8 dargestellten Konfiguration liegen radial äußere Flächen der Riegel 76 an der inneren Oberfläche der Ausnehmung in der Handhabungseinrichtung 18 und radial innere Oberflächen der Riegel 76 an den Abflachungen 68 am metallischen Bauteil 67 des Innenschafts 60 an. Dadurch sind radiale Bewegungen der Riegel 76 und eine Rotation des Innenschafts 60 um die Längsachse 28 relativ zum Außenschaft 70 formschlüssig unterbunden. Dadurch sind gleichzeitig die oben anhand der Figur 4 dargestellte mechanische Verbindung des distalen Endes 62 des Innenschafts 60 mit der Schneideeinrichtung 50 und die oben anhand der Figur 5 dargestellte mechanische Verbindung des Außenschafts 70 mit der Greifeinrichtung 30 verriegelt. Der Außenschaft 70 und der Innenschaft 60 können jedoch gemeinsam um die Längsachse 28 rotiert werden.

Somit nehmen die Riegel 76 in der Darstellung in Figur 7 entriegelnde Positionen und in der Darstellung in Figur 8 verriegelnde Positionen ein.

Die in axialer Richtung bzw. parallel zur Längsachse 28 gemessene Länge der Riegel 76 ist kürzer, insbesondere deutlich kürzer als die Länge der in Figur 6 gezeigten Abflachung 68 am metallischen Bauteil 67. Dadurch ist eine Relativbewegung von Innenschaft 60 und Außenschaft 70 in axialer Richtung möglich.

Abweichend von den Darstellungen in den Figuren 6 bis 8 können das metallische Bauteil 67 lediglich eine Abflachung 68 aufweisen und im Außenschaft 70 lediglich ein Riegel 76 vorgesehen sein.

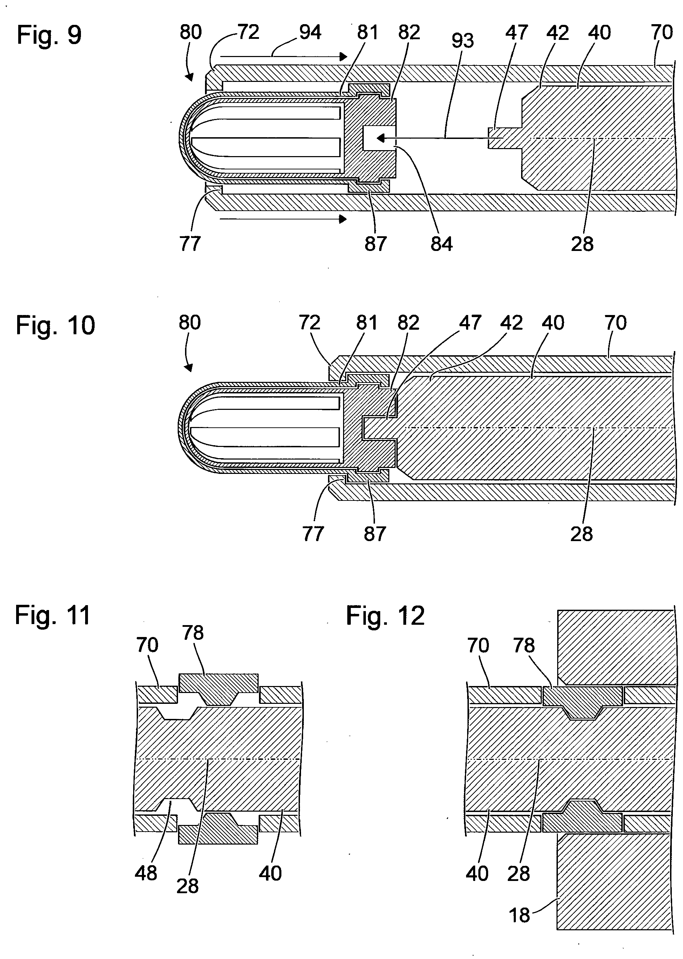

Figur 9 zeigt eine schematische Schnittdarstellung eines weiteren Werkzeugs 80. Die Schnittebene der Figur 9 enthält die Längsachse 28 (vgl. Figur 1). Das Werkzeug 80 weist anders als in Figur 1 angedeutet und im Unterschied zu dem Beispiel der Figuren 2 bis 8, keine schwenkbaren Maulteile und keine axial bewegbare Schneideeinrichtung auf. Stattdessen umfasst das Werkzeug 80 einen äußeren, käfigförmigen ersten Bestandteil 81 und einen inneren, ebenfalls käfigförmigen zweiten Bestandteil 82. Der zweite Bestandteil 82 ist relativ zum ersten Bestandteil 81 um die Längsachse 28 rotierbar, um Gewebe, das durch Öffnungen in den Bestandteilen 81, 82 ragt, abzuscheren.

Das Werkzeug 80 weist nahe seinem proximalen Ende am ersten Bestandteil 81 einen nach außen ragenden Kragen 87 und am zweiten Bestandteil 82 eine Ausnehmung 84 auf. Der Kragen 87 und die Ausnehmung 84 sind jeweils nicht rotationssymmetrisch zur Längsachse 28.

Ein Außenschaft 70 weist an seinem distalen Ende 72 einen nach innen ragenden Kragen 77 mit einer zum Kragen 87 am ersten Bestandteil 81 des Werkzeugs 80 korrespondierenden Gestalt auf. Eine Übertragungsstange 40 weist an ihrem distalen Ende 42 eine Kupplung 47 mit einer zur Ausnehmung 84 am zweiten Bestandteil 82 des Werkzeugs 80 korrespondierenden Gestalt auf.

Figur 10 zeigt eine weitere schematische Schnittdarstellung des Werkzeugs 80, der Übertragungsstange 40 und des Außenschafts 70. Die Darstellung in Figur 10 unterscheidet sich von der Darstellung in Figur 9 insbesondere dadurch, dass die Übertragungsstange 40 in der in Figur 9 angedeuteten ersten Richtung 93 und der Außenschaft 70 in der in Figur 9 angedeuteten zweiten Richtung 94 relativ zum Werkzeug 80 verschoben sind.

Durch eine Bewegung der Übertragungsstange 40 in der ersten Richtung 93 relativ zum Werkzeug 80 wird die Kupplung 47 am distalen Ende 42 der Übertragungsstange 40 in die Ausnehmung 84 am zweiten Bestandteil 82 des Werkzeugs 80 eingeführt. Wie bereits erwähnt weisen die Ausnehmung 84 am zweiten Bestandteil 82 des Werkzeugs 80 und die Kupplung 47 am distalen Ende 42 der Übertragungsstange 40 korrespondierende Querschnitte auf, die nicht rotationssymmetrisch zur Längsachse 28 sind. Dadurch sind die Übertragungsstange 40 und der zweite Bestandteil 82 des Werkzeugs 80 in der in Figur 10 gezeigten Konfiguration dahin gehend gekoppelt, dass eine Rotation der Übertragungsstange 40 um die Längsachse 28 mit einer entsprechenden Rotation des zweiten Bestandteils 82 des Werkzeugs 80 einhergeht.

Der Außenschaft 70 wird durch eine Bewegung in der zweiten Richtung 94, die zur ersten Richtung 93 entgegengesetzt ist, relativ zum Werkzeug 80 mit diesem verbunden. Wie bereits erwähnt sind der nach innen ragende Kragen 77 am distalen Ende 72 des Außenschafts 70 und der nach außen ragende Kragen 87 am ersten Bestandteil 81 des Werkzeugs 80 jeweils nicht rotationssymmetrisch zur Längsachse 28. Deshalb sind der Außenschaft 70 und der erste Bestandteil 81 des Werkzeugs 80 in der in Figur 10 gezeigten Konfiguration hinsichtlich einer Rotation um die Längsachse 28 miteinander gekoppelt.

Figur 11 zeigt eine schematische Schnittdarstellung des Außenschafts 70 und der Übertragungsstange 40 aus den Figuren 9 und 10 nahe deren proximalen Enden. Die Schnittebene der Figur 11 enthält die Längsachse 28 und entspricht den Schnittebenen der Figuren 9 und 10. Der Außenschaft 70 umfasst in zwei einander gegenüberliegenden Ausnehmungen je einen Riegel 78, der in radialer Richtung bewegbar ist. Die Übertragungsstange 40 weist eine ringförmig umlaufende Nut 48 auf. Bei der in Figur 11 gezeigten Konfiguration liegen die Riegel 78 außen an der Mantelfläche der Übertragungsstange 40 außerhalb der Nut 48 an.

Figur 12 zeigt eine weitere schematische Schnittdarstellung des Außenschafts 70 und der Übertragungsstange 40. Die Darstellung in Figur 12 entspricht hinsichtlich der dargestellten Schnittebene und des gewählten Ausschnitts der Darstellung in Figur 11. Die Übertragungsstange 40 ist jedoch gegenüber der Darstellung in Figur 11 relativ zum Außenschaft 70 so weit verschoben, dass die Riegel 78 nach radial innen verschoben in die Nut 48 an der Übertragungsstange 40 eingreifen. Dadurch schließen die Riegel 78 mit der äußeren Mantelfläche des Außenschafts 70 bündig ab, und die gesamte Anordnung aus Außenschaft 70 und Übertragungsstange 40 kann in eine Ausnehmung korrespondierenden Querschnitts in einer Handhabungseinrichtung 18 eingeführt werden.

Bei der in Figur 12 dargestellten Konfiguration liegen radial äußere Oberflachen der Riegel 78 an der inneren Oberfläche der Ausnehmung in der Handhabungseinrichtung 18 und radial innere Oberflächen der Riegel 78 an der Übertragungsstange 40, insbesondere an deren Oberfläche im Bereich der Nut 48 an. Dadurch sind radiale Bewegungen der Riegel 78 und eine axiale Verschiebung der Übertragungsstange 40 relativ zum Außenschaft 70 formschlüssig ausgeschlossen. Die Übertragungsstange 40 ist also relativ zum Außenschaft 70 hinsichtlich einer axialen Verschiebung verriegelt. Eine Rotation der Übertragungsstange 40 relativ zum Außenschaft 70 ist jedoch ebenso möglich wie eine gemeinsame axiale Translation von Übertragungsstange 40 und Außenschaft 70 zusammen.

Somit nehmen die Riegel 78 in der Darstellung in Figur 11 entriegelnde Positionen und in der Darstellung in Figur 12 verriegelnde Positionen ein.

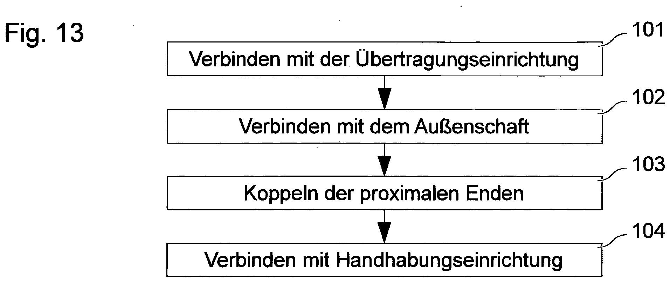

Figur 13 zeigt ein schematisches Flussdiagramm eines Verfahrens zum Zusammensetzen eines Werkzeugs, einer Übertragungseinrichtung und eines Außenschafts zu einem medizinischen Instrument. Das Verfahren ist auch mit Werkzeugen, Übertragungseinrichtungen und Außenschäften ausführbar, die sich von den oben anhand der Figuren 1 bis 12 dargestellten unterscheiden. Trotzdem werden nachfolgend beispielhaft Bezugszeichen aus den Figuren 1 bis 12 verwendet, um das Verständnis zu vereinfachen.

Bei einem ersten Schritt 101 werden ein Werkzeug 30, 50, 80 und eine Übertragungseinrichtung 40, 60 verbunden, wobei das Verbinden ein Bewegen der Übertragungseinrichtung 40, 60 relativ zum Werkzeug 30, 50, 80 in einer ersten Richtung 91, 93 umfasst. Bei einem zweiten Schritt 102 werden das Werkzeug 30, 50, 80 und eine Außenschaft 70 verbunden, wobei das Verbinden ein Bewegen des Außenschafts 70 relativ zum Werkzeug 30, 50, 80 in einer zweiten Richtung 92, 94, die entgegengesetzt zur ersten Richtung 91, 93 ist, umfasst. Bei einem dritten Schritt 103 werden das proximale Ende des Außenschafts 70 und das proximale Ende 61 der Übertragungseinrichtung 40, 60 derart gekoppelt, dass der Außenschaft 70 und die Übertragungseinrichtung 40, 60 nicht parallel zur ersten und zur zweiten Richtung relativ zueinander bewegbar sind. Der dritte Schritt 103 umfasst insbesondere eine Bewegung von einem oder mehreren Riegeln 76, 78 in radialen Richtungen von entriegelnden Positionen in verriegelnde Positionen. Bei einem vierten Schritt 104 wird das proximale Ende des Außenschafts 70 mit einer Handhabungseinrichtung 18 mechanisch verbunden. Dabei werden insbesondere die Riegel 76, 78 in ihren verriegelnden Positionen formschlüssig fixiert.

### Bezugszeichen

- **10**: **medizinisches Instrument**
- 11: proximales Ende des medizinischen Instruments 10
- 12: distales Ende des medizinischen Instruments 10
- 18: Handhabungseinrichtung am proximalen Ende 11 des medizinischen Instruments 10
- 19: Verriegelungseinrichtung an der Handhabungseinrichtung 18
- 20: Schaft des medizinischen Instruments 10
- 21: proximales Ende des Schafts 20
- 22: distales Ende des Schafts 20
- 28: Längsachse des Schafts 20
- **30**: **Greifeinrichtung** am distalen Ende 12 des medizinischen Instruments 10
- 31: proximales Ende der Greifeinrichtung 30
- 32: feststehendes Maulteil der Greifeinrichtung 30
- 34: schwenkbares Maulteil der Greifeinrichtung 30
- 37: Knagge am proximalen Ende 31 der Greifeinrichtung 30
- **40**: **Übertragungsstange** des medizinischen Instruments 10
- 42: distales Ende der Übertragungsstange 40
- 45: Nut in der Übertragungsstange 40
- 46: Isoliermantel an der Übertragungsstange 40
- 47: Kupplung am distalen Ende 42
- 48: umfängliche Nut in der Übertragungsstange 40
- **50**: **Schneideeinrichtung** am distalen Ende 12 des medizinischen Instruments 10
- 51: proximales Ende der Schneideeinrichtung 50
- 52: distales Ende der Schneideeinrichtung 50
- 53: Schneide an der Schneideeinrichtung 50
- 56: Vorsprung am proximalen Ende 51 der Schneideeinrichtung 50
- **60**: **Innenschaft** des medizinischen Instruments 10
- 61: proximales Ende des Innenschafts 60
- 62: distales Ende des Innenschafts 60
- 63: axialer Abschnitt eines L-förmigen Schlitzes am distalen Ende 62
- 64: umfänglicher Abschnitt eines L-förmigen Schlitzes am distalen Ende 62
- 65: Kupplungsbauteil am proximalen Ende 61 des Innenschafts 60
- 66: Isolierbauteil am Innenschaft 60
- 67: metallisches Bauteil des Innenschafts 60
- 68: Abflachung am metallischen Bauteil 67 des Innenschafts 60
- 69: Isoliermantel am Innenschaft 60
- **70**: **Außenschaft** des medizinischen Instruments 10
- 72: distales Ende des Außenschafts 70
- 73: axialer Abschnitt eines L-förmigen Schlitzes am distalen Ende 72
- 74: umfänglicher Abschnitt eines L-förmigen Schlitzes am distalen Ende 72
- 76: Riegel im Außenschaft 70
- 77: nach innen ragender Kragen am distalen Ende 72 des Außenschafts 70
- 78: Riegel im Außenschaft 70
- **80**: **Werkzeug**
- 81: erster Bestandteil des Werkzeugs 80
- 82: zweiter Bestandteil des Werkzeugs 80
- 84: Ausnehmung am zweiten Bestandteil des Werkzeugs 80
- 87: nach außen ragender Kragen am ersten Bestandteil 81 des Werkzeugs 80
- 91: erste Richtung
- 92: zweite Richtung
- 93: erste Richtung
- 94: zweite Richtung
- **101**: **erster Schritt** (Verbinden der Übertragungseinrichtung)
- 102: zweiter Schritt (Verbinden des Außenschafts)
- 103: dritter Schritt (Verbinden mit Übertragungseinrichtung)
- 104: vierter Schritt (Koppeln der proximalen Enden)

## Patentansprüche

1. Medizinisches Instrument (10), mit:
einem Außenschaft (70);
einer im Außenschaft (70) anzuordnenden Übertragungseinrichtung (40; 60) zum Übertragen zumindest entweder einer Kraft oder eines Drehmoments;
einem Werkzeug (30, 50; 80) mit einer Außenschaft-Kupplungseinrichtung (37, 87) zum lösbaren mechanischen Verbinden des Werkzeugs (30, 50; 80) mit einer Werkzeug-Kupplungseinrichtung (73, 74; 77) am distalen Ende (72) des Außenschafts (70) und mit einer Übertragungs-Kupplungseinrichtung (56; 84) zum lösbaren mechanischen Verbinden des Werkzeugs (30, 50; 80) mit einer Werkzeug-Kupplungseinrichtung (63, 64; 47) am distalen Ende (62) der Übertragungseinrichtung (40; 60),
wobei die mechanischen Verbindung der Werkzeug-Kupplungseinrichtung (73, 74; 77) am Außenschaft (70) mit der Außenschaft-Kupplungseinrichtung (37, 87) am Werkzeug (30, 50; 80) durch eine Bewegung des Außenschafts (70) in einer ersten Richtung (92; 94) relativ zum Werkzeug (30, 50; 80) herstellbar ist,
wobei die mechanischen Verbindung der Werkzeug-Kupplungseinrichtung (63, 64; 47) an der Übertragungseinrichtung (40; 60) mit der Übertragungs-Kupplungseinrichtung (56; 84) am Werkzeug (30, 50; 80) durch eine Bewegung der Übertragungseinrichtung (40; 60) in einer zweiten Richtung (91; 93) relativ zum Werkzeug (30, 50; 80) herstellbar ist,
**dadurch gekennzeichnet, dass** die erste Richtung (92; 94) entgegengesetzt zur zweiten Richtung (91; 93) ist.

2. Medizinisches Instrument (10) nach dem vorangehenden Anspruch, bei dem
die mechanischen Verbindung der Werkzeug-Kupplungseinrichtung (73, 74) am Außenschaft (70) mit der Außenschaft-Kupplungseinrichtung (37) am Werkzeug (30, 50) durch eine Rotation des Außenschaft (70) in einer ersten Richtung (92) relativ zum Werkzeug (30, 50) herstellbar ist,
die mechanischen Verbindung der Werkzeug-Kupplungseinrichtung (63, 64) an der Übertragungseinrichtung (40, 60) mit der Übertragungs-Kupplungseinrichtung (56) am Werkzeug (30, 50) durch eine Rotation der Übertragungseinrichtung (60) in einer zweiten Richtung (91) relativ zum Werkzeug (30, 50) herstellbar ist.

3. Medizinisches Instrument (10) nach einem der vorangehenden Ansprüche, bei dem zumindest entweder die Außenschaft-Kupplungseinrichtung (37) am Werkzeug (30, 50) und die Werkzeug-Kupplungseinrichtung (73, 74) am Außenschaft (70) oder die Übertragungs-Kupplungseinrichtung (56) am Werkzeug (30, 50) und die Werkzeug-Kupplungseinrichtung (63, 64) an der Übertragungseinrichtung (60) Bajonettkupplungen umfassen.

4. Medizinisches Instrument (10) nach einem der vorangehenden Ansprüche, bei dem die Übertragungseinrichtung einen Innenschaft (60) umfasst.

5. Medizinisches Instrument (10) nach dem vorangehenden Anspruch, ferner mit einer Übertragungsstange (40) zum Übertragen zumindest entweder einer Kraft oder eines Drehmoments, wobei die Übertragungsstange (40) zum Steuern einer ersten Funktion und der Innenschaft (60) zum Steuern einer zweiten Funktion ausgebildet ist.

6. Medizinisches Instrument (10) nach dem vorangehenden Anspruch, bei dem
die Übertragungsstange (40) mit einem schwenkbaren Maulteil (34) des Werkzeugs (30, 50) gekoppelt ist,
der Innenschaft (60) für eine mechanische Verbindung mit einer Schneideeinrichtung (50) des Werkzeug ausgebildet ist.

7. Medizinisches Instrument (10) nach dem vorangehenden Anspruch, bei dem
die Übertragungs-Kupplungseinrichtung am Werkzeug (30, 50) einen Vorsprung (56), der in einer Richtung senkrecht zur vorgesehenen Bewegungsrichtung der Schneideeinrichtung vorsteht, umfasst,
die Werkzeug-Kupplungseinrichtung an der Übertragungseinrichtung (40, 60) einen L-förmigen Schlitz (63, 64) oder eine L-förmige Nut zur Aufnahme des Vorsprungs (56) umfasst.

8. Medizinisches Instrument (10) nach einem der Ansprüche 6 und 7, bei dem
das Werkzeug (30, 50) zwei voneinander elektrisch isolierte Maulteile (32, 34) aufweist,
ein erstes der zwei voneinander elektrisch isolierten Maulteile (32, 34) mit einer Übertragungsstange (40) elektrisch leitfähig verbunden ist,
ein zweites der zwei voneinander elektrisch isolierten Maulteile (32, 34) mit dem Außenschaft (70) elektrisch leitfähig verbunden ist.

9. Medizinisches Instrument (10) nach dem vorangehenden Anspruch, bei dem der Innenschaft (60) einen distalen Abschnitt und einen vom distalen Abschnitt elektrisch isolierten proximalen Abschnitt (65) aufweist.

10. Medizinisches Instrument (10) nach einem der vorangehenden Ansprüche, ferner mit:
einer Handhabungseinrichtung (18) zur lösbaren mechanischen Verbindung mit den proximalen Enden (71, 41; 61) des Außenschafts (70) und der Übertragungseinrichtung (40; 60),
wobei die Handhabungseinrichtung (18), der Außenschaft (70) und die Übertragungseinrichtung (40; 60) so ausgebildet sind, dass der Außenschaft (70) und die Übertragungseinrichtung (40; 60) nicht parallel zur ersten und zur zweiten Richtung (91, 92; 93, 94) relativ zueinander bewegbar sind, wenn sie mit der Handhabungseinrichtung (18) in einer vorgesehenen Weise mechanisch verbunden sind.

11. Medizinisches Instrument (10) nach dem vorangehenden Anspruch, bei dem
der Außenschaft (70) einen Riegel (76) umfasst, der zwischen einer entriegelnden Position und einer verriegelnden Position bewegbar und ausgebildet ist, um in der verriegelnden Position in eine korrespondierende Ausnehmung (68) an der Übertragungseinrichtung (40; 60) einzugreifen,
die Handhabungseinrichtung (18) ausgebildet ist, um den Riegel (76) in der verriegelnden Position zu halten, wenn der Außenschaft (70) mit der Handhabungseinrichtung (18) mechanisch verbunden ist.

12. Medizinisches Instrument (10) nach einem der Ansprüche 10 und 11, bei dem Außenschaft (70) und Innenschaft (60) gemeinsam in Richtung parallel zur ersten und zweiten Richtung (91, 92; 93; 94) relativ zur Handhabungseinrichtung (18) bewegbar sind, wenn sie mit der Handhabungseinrichtung (18) mechanisch verbunden sind.

13. Verfahren zum Zusammensetzen eines Werkzeugs (30, 50; 80), einer Übertragungseinrichtung (40; 60) zum Übertragen zumindest entweder einer Kraft oder eines Drehmoments und eines Außenschafts (70) zu einem medizinischen Instrument (10) entsprechend einem der vorhergehenden Ansprüche 1- 12, mit folgenden Schritten:
mechanisches Verbinden (101) der Übertragungseinrichtung (40; 60) mit dem Werkzeug (30, 50; 80), wobei das Verbinden (101) ein Bewegen der Übertragungseinrichtung (40; 60) in einer ersten Richtung (91; 93) relativ zum Werkzeug (30, 50; 80) umfasst;
mechanisches Verbinden (102) des Außenschafts (70) mit dem Werkzeug (30, 50; 80), wobei das Verbinden (102) ein Bewegen des Außenschafts (70) in einer zweiten Richtung (92; 94) relativ zum Werkzeug (30, 50; 80) umfasst;
**dadurch gekennzeichnet, dass** die erste Richtung (91; 93) entgegengesetzt zur zweiten Richtung (92; 94) ist.

14. Verfahren nach dem vorangehenden Anspruch, ferner mit folgendem Schritt:
Koppeln (104) des proximalen Endes (71) des Außenschafts (70) und des proximalen Endes (61) der Übertragungseinrichtung (40; 60) derart, dass der Außenschaft (70) und die Übertragungseinrichtung (40; 60) nicht parallel zur ersten und zur zweiten Richtung (91, 92, 93, 94) relativ zueinander bewegbar sind.

15. Verfahren nach dem vorangehenden Anspruch, ferner mit folgendem Schritt:
mechanisches Verbinden (103) des proximalen Endes (71, 61,) des Außenschafts (70) mit einer Handhabungseinrichtung (18), wobei der Schritt des Koppelns (104) während des Schritts des Verbindens (103) der proximalen Enden erfolgt.

## Claims

1. Medical instrument (10), having:
an outer shaft (70);
a transmitting device (40; 60) to be arranged in the outer shaft (70) for transmitting at least either a force or a torque;
a tool (30, 50; 80) having an outer shaft coupling device (37, 87) for the detachable mechanical connection of the tool (30, 50; 80) and a tool coupling device (73, 74; 77) on the distal end (72) of the outer shaft (70) and having a transmitting coupling device (56; 84) for the detachable mechanical connection of the tool (30, 50; 80) and a tool coupling device (63, 64; 47) on the distal end (62) of the transmitting device (40; 60),
wherein the mechanical connection of the tool coupling device (73, 74; 77) on the outer shaft (70) and the outer shaft coupling device (37, 87) on the tool (30, 50; 80) is buildable by means of a movement of the outer shaft (70) in relation to the tool (30, 50; 80) in a first direction (92; 94),
wherein the mechanical connection of the tool coupling device (63, 64; 47) on the transmitting device (40; 60) and the transmitting coupling device (56; 84) on the tool (30, 50; 80) is buildable by means of a movement of the transmitting device (40; 60) in relation to the tool (30, 50; 80) in a second direction (91; 93),
**characterized in that** the first direction (92; 94) is opposite to the second direction (91; 93).

2. Medical instrument (10) according to the preceding claim, where
the mechanical connection of the tool coupling device (73, 74) on the outer shaft (70) and the outer shaft coupling device (37) on the tool (30, 50) is buildable by means of a rotation of the outer shaft (70) in relation to the tool (30, 50) in a first direction (92),
the mechanical connection of the tool coupling device (63, 64) on the transmitting device (40, 60) and the transmitting coupling device (56) on the tool (30, 50) is buildable by means of a rotation of the transmitting device (60) in relation to the tool (30, 50) in a second direction (91).

3. Medical instrument (10) according to one of the preceding claims, where at least either the outer shaft coupling device (37) on the tool (30, 50) and the tool coupling device (73, 74) on the outer shaft (70) or the transmitting coupling device (56) on the tool (30, 50) and the tool coupling device (63, 64) on the transmitting device (60) include bayonet couplings.

4. Medical instrument (10) according to one of the preceding claims, where the transmitting device includes an inside shaft (60).

5. Medical instrument (10) according to the preceding claim, additionally having a transmitting rod (40) for transmitting at least either a force or a torque, wherein the transmitting rod (40) is realized for controlling a first function and the inside shaft (60) is realized for controlling a second function.

6. Medical instrument (10) according to the preceding claim, where
the transmitting rod (40) is coupled with a pivotable mouth part (34) of the tool (30, 50),
the inside shaft (60) is realized for a mechanical connection to a cutting device (50) of the tool.

7. Medical instrument (10) according to the preceding claim, where
the transmitting coupling device on the tool (30, 50) includes a projection (56) which protrudes in a direction at right angles with respect to the provided direction of movement of the cutting device,
the tool coupling device on the transmitting device (40, 60) includes an L-shaped slot (63, 64) or an L-shaped groove for accommodating the projection (56).

8. Medical instrument (10) according to either of Claims 6 and 7, where
the tool (30, 50) has two mouth parts (32, 34) which are electrically insulated from one another,
a first of the two mouth parts (32, 34) which are electrically insulated from one another is connected to a transmitting rod (40) so as to be electrically conductive,
a second of the of the two mouth parts (32, 34) which are electrically insulated from one another is connected to the outer shaft (70) so as to be electrically conductive.

9. Medical instrument (10) according to the preceding claim, where the inside shaft (60) has a distal portion and a proximal portion (65) which is electrically insulated from the distal portion.

10. Medical instrument (10) according to one of the preceding claims, additionally having:
a handling device (18) for the detachable mechanical connection to the proximal ends (71, 41; 61) of the outer shaft (70) and of the transmitting device (40; 60),
wherein the handling device (18), the outer shaft (70) and the transmitting device (40; 60) are realized such that the outer shaft (70) and the transmitting device (40; 60) are not movable in relation to one another in a parallel manner with respect to the first and to the second direction (91, 92; 93, 94) when they are mechanically connected to the handling device (18) in a provided manner.

11. Medical instrument (10) according to the preceding claim, where
the outer shaft (70) includes a locking bar (76) which is movable between an unlocking position and a locking position and is realized in order to engage in a corresponding recess (68) on the transmitting device (40; 60) in the locking position,
the handling device (18) is realized in order to hold the locking bar (76) in the locking position when the outer shaft (70) is mechanically connected to the handling device (18).

12. Medical instrument (10) according to either of claims 10 and 11, where the outer shaft (70) and the inside shaft (60) are movable together in relation to the handling device (18) in the direction parallel to the first and second direction (91, 92; 93; 94) when they are mechanically connected to the handling device (18).

13. Method for fitting together a tool (30, 50; 80), a transmitting device (40; 60) for transmitting at least either a force or a torque and an outer shaft (70) to form a medical instrument (10) corresponding to one of the preceding Claims 1-12, said method having the following steps:
mechanically connecting (101) the transmitting device (40; 60) to the tool (30, 50; 80), wherein the connecting process (101) includes moving the transmitting device (40; 60) in relation to the tool (30, 50; 80) in a first direction (91; 93);
mechanically connecting (102) the outer shaft (70) to the tool (30, 50; 80), wherein the connecting process (102) includes moving the outer shaft (70) in relation to the tool (30, 50; 80) in a second direction (92; 94);
**characterized in that** the first direction (91; 93) is opposite to the second direction (92; 94).

14. Method according to the preceding claim, said method additionally having the following step:
coupling (104) the proximal end (71) of the outer shaft (70) and the proximal end (61) of the transmitting device (40; 60) in such a manner that the outer shaft (70) and the transmitting device (40; 60) are not movable in relation to one another in a parallel manner with respect to the first and to the second direction (91, 92, 93, 94).

15. Method according to the preceding claim, said method additionally having the following step:
mechanically connecting (103) the proximal end (71, 61) of the outer shaft (70) to a handling device (18), wherein the step of coupling (104) is effected during the step of connecting (103) the proximal ends.

## Revendications

1. Instrument médical (10), avec :
- une tige externe (70) ;
- un mécanisme de transmission (40 ; 60) devant être disposé dans la tige externe (70) en vue de la transmission de tout au moins, soit une force, soit un couple ;
- un outil (30, 50 ; 80) avec un mécanisme de couplage (37, 87) de la tige externe en vue de la jonction mécanique, de type amovible, de l'outil (30, 50 ; 80) avec un mécanisme de couplage de l'outil (73, 74 ; 77) au niveau de l'extrémité distale (72) de la tige externe (70), et avec un mécanisme de couplage de la transmission (56 ; 84) en vue de la jonction mécanique, de type amovible, de l'outil (30, 50 ; 80) avec un mécanisme de couplage de l'outil (63, 64 ; 47) au niveau de l'extrémité distale (62) du mécanisme de transmission (40 ; 60) ;
dans lequel la jonction mécanique du mécanisme de couplage de l'outil (73, 74 ; 77) situé au niveau de la tige externe (70) peut être établie avec le mécanisme de couplage (37, 87) de la tige externe situé au niveau de l'outil (30, 50 ; 80), par l'intermédiaire d'un mouvement de la tige externe (70) dans une première direction (92 ; 94) par rapport à l'outil (30, 50 ; 80) ;
dans lequel la jonction mécanique du mécanisme de couplage de l'outil (63, 64 ; 47) situé au niveau du mécanisme de transmission (40 ; 60) peut être établie avec le mécanisme de couplage de la transmission (56 ; 84) situé au niveau de l'outil (30, 50 ; 80), par l'intermédiaire d'un mouvement du mécanisme de transmission (40 ; 60) dans une deuxième direction (91 ; 93) par rapport à l'outil (30, 50 ; 80) ; **caractérisé en ce que**
la première direction (92 ; 94) est opposée à la deuxième direction (91 ; 93).

2. Instrument médical (10) selon la revendication qui précède, pour lequel
la jonction mécanique du mécanisme de couplage de l'outil (73, 74) situé au niveau de la tige externe (70) peut être établie avec le mécanisme de couplage (37) de la tige externe situé au niveau de l'outil (30, 50), par l'intermédiaire d'une rotation de la tige externe (70) dans une première direction (92) par rapport à l'outil (30, 50) ;
la jonction mécanique du mécanisme de couplage de l'outil (63, 64) situé au niveau du mécanisme de transmission (40, 60) peut être établie avec le mécanisme de couplage de la transmission (56) situé au niveau de l'outil (30, 50), par l'intermédiaire d'une rotation du mécanisme de transmission (60) dans une deuxième direction (91) par rapport à l'outil (30, 50).

3. Instrument médical (10) selon l'une des revendications qui précèdent, pour lequel tout au moins, soit le mécanisme de couplage (37) de la tige externe situé au niveau de l'outil (30, 50) et le mécanisme de couplage de l'outil (73, 74) situé au niveau de la tige externe (70), soit le mécanisme de couplage de la transmission (56) situé au niveau de l'outil (30, 50) et le mécanisme de couplage de l'outil (63, 64) situé au niveau du mécanisme de transmission (60) comprennent des raccords à baïonnette.

4. Instrument médical (10) selon l'une des revendications qui précèdent, pour lequel le mécanisme de transmission comprend un arbre intérieur (60).

5. Instrument médical (10) selon la revendication qui précède, comprenant en outre une bielle de transmission (40) destinée à la transmission de tout au moins, soit une force, soit un couple, dans lequel la bielle de transmission (40) est conçue en vue de la commande d'une première fonction et l'arbre intérieur (60) est conçu en vue de la commande d'une deuxième fonction.

6. Instrument médical (10) selon la revendication qui précède, pour lequel
la bielle de transmission (40) est couplée avec une partie de mâchoire (34) de l'outil (30, 50), laquelle peut pivoter ;
l'arbre intérieur (60) est conçu pour une jonction mécanique avec un mécanisme de coupe (50) de l'outil.

7. Instrument médical (10) selon la revendication qui précède, pour lequel
le mécanisme de couplage de la transmission situé au niveau de l'outil (30, 50) comprend une proéminence (56), laquelle fait saillie dans une direction qui se trouve à la perpendiculaire de la direction du mouvement prévue du mécanisme de coupe ;
le mécanisme de couplage de l'outil situé au niveau du mécanisme de transmission (40, 60) comprend une fente (63, 64) en forme de « L » ou une rainure en forme de « L » en vue de la réception de la proéminence (56).

8. Instrument médical (10) selon l'une des revendications 6 et 7, pour lequel
l'outil (30, 50) présente deux parties de mâchoire (32, 34), lesquelles sont isolées électriquement l'une de l'autre ;
une première parmi les deux parties de mâchoire (32, 34) isolées électriquement l'une de l'autre est raccordée de manière électriquement conductrice à une bielle de transmission (40) ;
une deuxième parmi les deux parties de mâchoire (32, 34) isolées électriquement l'une de l'autre est raccordée de manière électriquement conductrice à la tige externe (70).

9. Instrument médical (10) selon la revendication qui précède, pour lequel l'arbre intérieur (60) présente une section distale et une section proximale (65), laquelle est isolée électriquement de la section distale.

10. Instrument médical (10) selon l'une des revendications qui précèdent, comprenant en outre :
un mécanisme de manipulation (18) destiné à la jonction mécanique, de type amovible, avec les extrémités proximales (71, 41 ; 61) de la tige externe (70) et du mécanisme de transmission (40 ; 60) ;
dans lequel le mécanisme de manipulation (18), la tige externe (70) et le mécanisme de transmission (40 ; 60) sont conçus de telle sorte que la tige externe (70) et le mécanisme de transmission (40 ; 60) ne peuvent pas être déplacés, l'un par rapport à l'autre, parallèlement à la première et à la deuxième direction (91, 92 ; 93, 94), quand ils sont reliés de manière mécanique avec le mécanisme de manipulation (18) dans une manière prévue.

11. Instrument médical (10) selon la revendication qui précède, pour lequel
la tige externe (70) comprend un verrouillage (76), lequel peut être déplacé entre une position de déverrouillage et une position de verrouillage et lequel est conçu afin de venir se mettre en prise, dans la position de verrouillage, à l'intérieur d'une cavité (68) correspondante qui se trouve au niveau du mécanisme de transmission (40 ; 60) ;
le mécanisme de manipulation (18) est conçu afin de maintenir le verrouillage (76) dans la position de verrouillage, quand la tige externe (70) est reliée mécaniquement avec le mécanisme de manipulation (18).

12. Instrument médical (10) selon l'une des revendications 10 et 11, pour lequel la tige externe (70) et l'arbre intérieur (60) peuvent être déplacés ensemble dans la même direction parallèlement à la première et à la deuxième direction (91, 92 ; 93 ; 94), par rapport au mécanisme de manipulation (18), quand ils sont reliés de manière mécanique avec le mécanisme de manipulation (18).

13. Procédé destiné à l'assemblage d'un outil (30, 50 ; 80), d'un mécanisme de transmission (40 ; 60) destiné à la transmission de tout au moins, soit une force, soit un couple et d'une tige externe (70) vers un instrument médical (10), conformément à l'une des revendications qui précèdent 1 à 12,
lequel procédé comprend les phases suivantes :
- la jonction mécanique (101) du mécanisme de transmission (40 ; 60) avec l'outil (30, 50 ; 80), dans lequel la jonction (101) comprend un déplacement du mécanisme de transmission (40 ; 60) dans une première direction (91 ; 93) par rapport à l'outil (30, 50 ; 80) ;
- la jonction mécanique (102) de la tige externe (70) avec l'outil (30, 50 ; 80), dans lequel la jonction (102) comprend un déplacement de la tige externe (70) dans une deuxième direction (92 ; 94) par rapport à l'outil (30, 50 ; 80) ;
**caractérisé en ce que** la première direction (91 ; 93) est opposée à la deuxième direction (92 ; 94).

14. Procédé selon la revendication qui précède, lequel comprend en outre la phase suivante :
le couplage (104) de l'extrémité proximale (71) de la tige externe (70) et de l'extrémité proximale (61) du mécanisme de transmission (40 ; 60), de telle sorte que la tige externe (70) et le mécanisme de transmission (40 ; 60) ne peuvent pas être déplacés, l'un par rapport à l'autre, parallèlement à la première et à la deuxième direction (91, 92, 93, 94).

15. Procédé selon la revendication qui précède, lequel comprend en outre la phase suivante :
- la jonction mécanique (103) de l'extrémité proximale (71, 61) de la tige externe (70) avec un mécanisme de manipulation (18), dans lequel la phase de couplage (104) a lieu pendant la phase de la jonction (103) des extrémités proximales.
